(19) Europäisches Patentamt
European Patent Office
Office européen des brevets

(11) **EP 3 708 264 B1**

(12) **EUROPEAN PATENT SPECIFICATION**

(45) Date of publication and mention
of the grant of the patent:
**07.05.2025 Bulletin 2025/19**

(21) Application number: **19162705.8**

(22) Date of filing: **14.03.2019**

(51) International Patent Classification (IPC):
**B06B 1/06** *(2006.01)*     **G01N 29/28** *(2006.01)*
**A61B 8/00** *(2006.01)*     **G01N 29/06** *(2006.01)*

(52) Cooperative Patent Classification (CPC):
**B06B 1/0622; A61B 8/4281; A61B 8/4494;**
**G01N 29/0654; G01N 29/28;** G01N 2291/2634;
G01N 2291/2638

(54) **AN ACOUSTIC COUPLING INTERFACE**

AKUSTISCHE KOPPLUNGSSCHNITTSTELLE

INTERFACE DE COUPLAGE ACOUSTIQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**16.09.2020 Bulletin 2020/38**

(73) Proprietor: **IMEC vzw**
**3001 Leuven (BE)**

(72) Inventor: **ROTTENBERG, Xavier**
**3001 Leuven (BE)**

(74) Representative: **AWA Sweden AB**
**Box 45086**
**104 30 Stockholm (SE)**

(56) References cited:
**US-A- 5 680 863**     **US-A1- 2010 011 865**
**US-A1- 2019 046 158**

**Description**

Technical field

The present inventive concept relates to the field of ultrasonic examination.

More particularly it relates to an acoustic coupling interface for use with a flexible ultrasound transducer.

Background

Large 2D arrays of ultrasound arrays have several applications in the medical market and for consumer electronics. Examples are medical imaging, gesture recognition, directed sound, fingerprint detection and mid-air haptics.

The standard structure of a piezoelectric micromachined ultrasound transducer (pMUT) is known in the art. A small drum is made, with a suspended membrane on top of a small cavity. The dimensions of this cavity in combination with the stiffness of the membrane will determine the resonance frequency of a particular MUT. As an example, the MUT may be driven by the piezo-electric effect (pMUT). By applying an AC electric field at the resonance frequency across a piezoelectric material, a stress difference between the piezo-electric material and the membrane is generated, and this will induce a vibration and the emission of an acoustical wave. Typical frequencies are in the range of 50 kHz to 20 MHz. Applications that use beam-forming to create a focal spot in emission or to image a small spot in receiving, require larger arrays of ultrasound transducers working together.

There is a need in the art for improved designs of ultrasound transducers and systems for examining curved object, e.g. for imaging or for obtaining data representative of features of an objects, since ultrasound transducers that are manufactured on flat rigid substrates may not be fit for scanning curved objects. The operator has to move and press the flat transducer against the curved object to be examined, which leads to difficulties in reproduction of images etc.

Flexible ultrasound transducer is known from e.g. US 20180168544, which discloses methods and systems for coupling a flexible transducer to an object. A transducer positioning device includes an inflatable bladder and a strap. The inflatable bladder may apply a force to a transducer array to maintain its position against the object when inflated. The strap may hold the bladder against the transducer array. Once in place, the bladder may be inflated with a fluid.

However, there is a need in the art for solutions that allow for improved ultrasonic examination of curved objects using flexible ultrasound transducers.

US 2019/0046158 A1 discloses an ultrasound apparatus for medical examination of a subject. The ultrasound apparatus comprises a plurality of ultrasound transducers for emitting and receiving ultrasound waves and for providing different ultrasound signals on the basis of the ultrasound waves. A connection layer is provided, which is attachable to the subject, wherein the ultrasound transducers are coupled to the connection layer. A processing unit, which is connectable to the ultrasound transducers is provided for receiving the ultrasound signals and for determining at least one parameter on the basis of the ultrasound signals. The processing unit is adapted to determine at least one parameter indicative of a relative position of the ultrasound transducers to each other and/or a shape of the connection layer and the ultrasound waves are surface acoustic waves transmitted through the connection layer or a layer attached to the connection layer.

US 5680863 A1 discloses a phased array transducer for an ultrasonic imaging system includes a flexible support element which supports an array of piezoelectric transducer elements. Shape transducers such as strain gauges or capacitive transducers are coupled to the support element to generate a signal indicative of the instantaneously prevailing curvature of the array. A user-controlled actuator is coupled to the support element to flex the support element between at least first and second configurations, wherein the support element has separate curvatures along the axis of the transducer in each of the first and second configurations. In some applications the transducer elements are positioned for separate scan formats in the first and second configurations. The support element can be provided with first and second alternating regions which differ in flexibility, in order to concentrate flexing of the support element in the second regions. In some applications the support element may have a cross-sectional dimension of less than about ten millimeters to facilitate insertion of the transducer into a tortuous vessel of a living subject.

US 2010/0011865 A1 discloses an apparatus and method for testing composite structures in which ultrasonic waves are used to detect disbands in the structures are described. The apparatus comprises a flexible structure carrying acousto-optical transducers such as fiber Bragg gratings. During use, the apparatus is mechanically and conformally coupled to the structure under test.

Summary

It is an object of the invention to at least partly overcome one or more limitations of the prior art. In particular, it is an object to provide a coupling interface for a flexible ultrasound transducing device that provides for improved ultrasonic examination of curved objects.

As a first aspect of the invention, there is provided an acoustic coupling interface for use between a flexible ultrasound transducing device and a curved object to be examined; wherein said interface is in the form of a sheet having a bending flexibility that permits the sheet to form a continuous contact with said curved object during operation of the flexible ultrasound transducing device, and wherein said sheet comprises a bulk material and a plurality of acoustic waveguiding structures arranged in said bulk material, wherein the plurality of acoustic waveguiding structures is for providing bidirectional coupling of ultrasound signals emitted by the ultrasound transducing device.

The acoustic coupling interface may be a disposable interface and is used between a flexible ultrasound transducing device and a curved object to be examined. The object being examined refers to the object in contact with the acoustic coupling interface during examination using a flexible ultrasound transducing device. This object may thus be curved, even though data representative of features that are not curved but resides within the examined curved object is gathered during examination.

The curved objects suitable may be objects comprising a curvature with a curvature radius of less than 20 cm, such as less than 10 cm. The curved object may be a part of a body of a patient, such as an arm or a leg. The acoustic coupling interface is a flexible interface that may follow the flexing of a flexible ultrasound transducer during ultrasonic examination of a curved object.

The acoustic coupling interface is further in the form of a sheet. The sheet of the acoustic interface generally consists of two planar surfaces, the first and second planar surface, oppositely arranged of each other, i.e. having normal vectors pointing in two different and parallel directions.

The first and second planar surfaces may thus extend in an X-Y plane and the sheet may a thickness defined in a Z-direction that is perpendicular to both X and Y directions. The sheet has further a bending flexibility, e.g. a bending flexibility in the X-Y plane in positive or negative Z-direction, that allows the acoustic coupling interface to bend around curved object and for a continuous contact with the curved object without breaking during examination of the curved object. An imaginary line drawn between two points on the surface of the sheet, said points being separated in the Z direction, is not straight but curved when the sheet is being flexed or bent in the Z-direction.

The bulk material of the acoustic interface comprises a plurality of acoustic waveguiding structures. The sheet may thus consist of the bulk material.

The bulk material may itself be a flexible material.

The bulk material may comprise or consist of a rubber or a polymeric material. Thus, in embodiments of the first aspect, the bulk material comprises a polymer. The bulk material may be a rubber or comprise a rubber. Moreover, the bulk material may be selected from the group consisting of SU-8, silicon nitride and polyimide.

Further, the bulk material may be a layered material. Thus, the bulk material may comprise a multi-layered structure comprising individual layers stacked on top of each other. One such individual layer does not need to be flexible, but the whole multi-layered structure may be flexible. As an example, individual layers of the multi-layered structure may comprise or consist of silicon oxide.

The waveguiding structures may comprise acoustic waveguides, such as an array of acoustic waveguides. Furthermore, the acoustic waveguiding structures may comprise a set of acoustic scatterers arranged in the bulk material that together work or function as an acoustic waveguiding structure.

The plurality of acoustic waveguiding structures is for providing bidirectional coupling of ultrasound signals emitted by the ultrasound transducing device. Thus, the acoustic coupling interface facilitates transmittance of ultrasound signals emitted by a flexible ultrasound transducer into the object being examined and allows for the resultant echo signals from the object being transmitted back to the flexible ultrasound transducer. The acoustic waveguiding structures therefore function as an acoustic lens for the ultrasonic waves emitted by an ultrasonic transducer and for the echo signal received from the object being examined.

The first aspect of the invention is based on the insight that the flexible acoustic interface permits good acoustic contact between the object that is examined, such as a person, and a flexible ultrasound transducer, such as a flexible ultrasound transducer comprising an array of ultrasonic transducing elements. The flexible acoustic interface thus transfers the shape or curvature of the object being examined to the flexible ultrasound transducer. The flexible acoustic interface may further function as an alternative or complement to a gel that is conventionally used when performing ultrasonic examination of e.g. a human body. Due to the waveguiding structures, the acoustic interface couples acoustic waves in a way that allows nonuniform, and possibly non-sticking, contact with the object being examined.

Further, the acoustic interface may function as a disposable patch, which permits reuse of the flexible ultrasound transducer.

In embodiments of the first aspect, the sheet has a first planar surface arranged for contacting said flexible ultrasound transducing device and a second planar surface, opposite said first surface, arranged for contacting said curved object to be examined, and wherein said sheet has a bending flexibility such that the surface profiles of both the first and second planar surfaces are altered with the second planar surface conforming to the curved object when the sheet is in contact with said curved object during operation.

The flexibility and thickness of the sheet may such that the surface profiles of the first and second planar surfaces are affected during examination, i.e. when a flexible transducer is pressed against the object being examined. Consequently, this aids in "transferring" the curvature of the object to a flexible ultrasound transducer.

As an example, the first and second planar surfaces may have a length and width that both are at least five times the thickness of the sheet, such as at least 10 times, such as at least 20 times, such as at least 50 times, the thickness of the sheet. Thus, both the length of the sheet as well as the width of the sheet may be at least five times longer than the thickness.

As an example, the sheet may have a length and width that is at least 0.2 cm, such as at least 1 cm, such as at least 5 cm, such as at least 10 cm, such as at least 50 cm.

As an example, the sheet may have a surface area that is between 0.20 cm$^2$ and 0.50 cm$^2$. As a further example, the sheet may have a surface area that is between 1 cm$^2$ and 25 cm$^2$. As a further example, the sheet may have a surface area that is between 30 cm$^2$ and 70 cm$^2$.

As a further example, the sheet may have a surface area that is at least 80 cm$^2$, such as about 100 cm$^2$.

Moreover, the sheet may have a surface area that is more than 0.10 m$^2$, such as more than 0.20 m$^2$.

Further, the sheet may have a thickness that is between 10 $\mu$m and 1 cm, such as between 0.5 mm and 1.0 mm.

As an example, the first and second planar surfaces may extend in an X and Y direction and wherein the sheet has a thickness extending in a Z direction that is perpendicular to the X and Y directions. The sheet may have a flexibility such that the sheet may be bent in the Z direction with a bend angle that is at least 30 degrees, such as at least 45 degrees, such as at least 90 degrees.

In embodiments of the first aspect, the sheet has a bending flexibility that permits the sheet to be bent with a radius of curvature (Rc) that is less than 5 cm, preferably less than 3 cm, more preferably less than 2 cm.

In other embodiments of the first aspect, the sheet has a bending flexibility that permits the sheet to be bent with a radius of curvature (Rc) that is between 10 and 30 cm, such as between 15 and 20 cm.

The radius of curvature may be defined when bending in positive or negative Z-direction, i.e. the direction along the normal to the first and/or second planar surfaces. The minimum radius of curvature may be less than 5 cm, such as 1 - 4 cm.

Furthermore, in embodiments of the first aspect, the whole sheet or the bulk material has a flexural modulus (modulus of elasticity) that permits the sheet to form a continuous contact with said curved object during operation of the flexible ultrasound transducing device.

The flexural modulus of a material is a physical property denoting the ability for that material to bend. In mechanical terms, it is the ratio of stress to strain during a flexural deformation, or bending. If the sheet is made of plastics, the type of polymer, molecular weight and thickness may affect the flexibility.

As an example, the whole sheet or the bulk material may have a flexural modulus that is less than 50 GPa, such as less than 20 GPa, thereby permitting the sheet to form a continuous contact with said curved object during operation of the flexible ultrasound transducing device.

The flexural modulus of a material may be measured using a known 3-point analysis on a rectangular beam of the material having width w and height h. Parameter L defines a length between two support points on a first side of the beam and a force F is applied on the other side of the beam. The displacement or deflection d is measured and the flexural modulus, $E_{bend}$ (force per area) is calculated using

$$E_{bend} = (L^3 F) / (4wh^3 d)$$

In embodiments of the first aspect, the plurality of acoustic waveguiding structures are arranged in an array in the bulk material. The array may be one-dimensional, two-dimensional and/or three dimensional.

As an example, the plurality of acoustic waveguiding structures may comprise more than 20, such as more than 50, individual acoustic waveguiding structures or acoustic waveguides.

In embodiments of the first aspect, the sheet is a stretchable sheet. Thus, the sheet of the acoustic coupling interface may be of a material that can withstand strain reversibly. Thus, the sheet may also comprise or consist of an elastic material.

However, the sheet may also comprise or consist of a non-elastic material.

In the first aspect, the plurality of acoustic waveguiding structures comprises waveguides having an elongated form.

The waveguides are thus in the form of pillar extending through part or the whole bulk material.

In embodiments of the first aspect, the plurality of acoustic waveguiding structures comprises an arrangement of alternating bulk material and another material different from the bulk material, said arrangement extending from the first planar surface to the second planar surface.

As an alternative, the acoustic waveguiding structures may be entirely enclosed by the bulk material.

The material different from the bulk material may be voids or a material having a different acoustic impedance than the bulk material

The arrangement of alternating bulk material and said "another material" may form a three-dimensional array of discrete elements of said "another material" within the bulk material. The discrete elements, such as a row of discreet elements in the three-dimensional array, may function together as an acoustic waveguiding structure.

As an example, the plurality of acoustic waveguiding structures may comprises waveguides extending from the first planar surface to the second planar surface. Consequently, the acoustic waveguiding structure may extend in the whole Z-direction or through the whole thickness of the sheet. The waveguides may thus form a plurality of pillars extending within the bulk material though the thickness of the sheet in a direction substantially perpendicular to the first or second planar surface of the sheet.

In embodiments of the first aspect, the plurality of acoustic waveguiding structures comprises a solid material different than the bulk material.

The solid material different than the bulk material may have a different acoustic impedance than the bulk material. The solid material may be a metallic material or a polymeric material.

As an example, the solid material of the plurality of acoustic waveguiding structures may protrudes out from the first and/or second planar surface. Thus, the plurality of acoustic waveguiding structures may only protrudes out from the first planar surface of the sheet, they may protrude from both the first and second planar surface of the sheet or they may protrude only out from the second surface of the sheet. This may be advantageous in that the protruding waveguiding structures may facilitate adhesion to another surface during operation, such as to the surface of the curved object being examined or to the surface of a flexible ultrasound transducer. The protruding waveguiding structures of a solid material may thus form an array of protruding elements that increases the adhesive properties of the acoustic coupling interface.

In embodiments of the first aspect, the plurality of acoustic waveguiding structure structures comprises internal walls in the bulk material so as to define waveguiding structures in the form of voids in the bulk material. As discussed above, the voids may have an elongated form and extend from the first to the second planar surface of the sheet.

As a second aspect of the invention, there is provided, a system for creating data representative of features of a curved object comprising

a flexible ultrasound transducing device, and an acoustic coupling interface according to the first aspect above configured to be removably attached to said ultrasound transducing device such that ultrasound signals emitted by the transducing device are transmitted into said object and resultant echo signals from the object are transmitted back to the ultrasound transducing device.

This aspect may generally present the same or corresponding advantages as the former aspect.

The data representative of features of a curved object that is generated may be used for imaging of the curved object.

The flexible ultrasound transducer may comprise an array of ultrasound transducing elements. The ultrasound transducing elements may be configured for generating ultrasonic energy propagating along a main transducer axis (Z). The flexible ultrasound transducing device may comprise a first outer surface facing said curved object during examination and having a normal vector that is parallel to the main transducer axis. The acoustic coupling interface may thus be configured to be removably attached to such a first outer surface of the flexible ultrasound transducer. The first outer surface of the ultrasound transducer may have a surface area that is at least $100\,cm^2$, such as at least $400\,cm^2$. Thus, the array of transducing elements of such a flexible ultrasound transducer may cover an area that is at least $100\,cm^2$, such as at least $400\,cm^2$.

The system may thus be provided as a kit with a flexible ultrasound transducer and at least one acoustic coupling interface according to the first aspect above. The flexible ultrasound transducer may be reused whereas the acoustic coupling interface may be a disposable interface that is changed between ultrasonic examinations. The system is advantageous e.g. in that provides for ultrasonic examination, such as imaging, without having to use a traditional gel for acoustic coupling between the object being examined and the ultrasound transducer.

As a third aspect of the invention, there is provided a method of obtaining data representative of features of an object comprising

- subjecting the object to ultrasound signals using a system according to the second aspect above; and
- analysing the resultant echo signals from the object and thereby obtaining data representative of features of said object based on the resultant echo signals.

This aspect may generally present the same or corresponding advantages as the former aspects discussed above. The object may be a curved object, such as a curved object comprising a curvature having a curvature with a curvature radius of less than 20 cm, such as less than 10 cm. The curved object may be a part of a body of a patient, such as an arm or a leg

The step of analysing the resultant echo signals may comprise forming an image of a part of the object being examined, such as the inside of the object being examined.

In embodiments of the third aspect, the step of subjecting the object to ultrasound signals is performed with a direct contact of the acoustic coupling interface and the object and/or a direct contact of the acoustic coupling interface and the flexible ultrasound transducing device.

As an example, the step of subjecting the object to ultrasound signals may be performed with a direct contact of the acoustic coupling interface and the object, e.g. without any gel between the object and the acoustic coupling interface.

As a further example, the step of subjecting the object to ultrasound signals may performed with a direct contact of the acoustic coupling interface and the flexible ultrasound transducing device, e.g. without any gel between the acoustic coupling interface and the flexible ultrasound transducing device.

As another example, the step of subjecting the object to ultrasound signals may performed with a direct contact of the acoustic coupling interface and the flexible ultrasound transducing device and a direct contact of the acoustic coupling interface and the object, e.g. without any gel between the acoustic coupling interface and the flexible ultrasound transducing device and without any gel between the object and the acoustic coupling interface.

However, the step of subjecting the object to ultrasound signals may as an alternative be performed with an indirect contact of the acoustic coupling interface and the object and/or an indirect contact of the acoustic coupling interface and the flexible ultrasound transducing device. Thus, a gel may be used between the acoustic coupling interface and the object or between the acoustic coupling interface and the flexible ultrasound transducing device. As a further example, a gel may be used between the acoustic coupling interface and the object as well as between the acoustic coupling interface and the flexible ultrasound transducing device.

Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.

Fig. 1 is a perspective view of a schematic illustration of an acoustic coupling interface of the present disclosure.

Figs. 2a-f are illustrative embodiments of the acoustic waveguiding structures arranged in the bulk material of the sheet.

Fig. 3 is a schematic illustration of the bend angle and the radius of curvature when the acoustic coupling interface is bent in negative Z-direction.

Figs 4a-d shows illustrative embodiments of a system for creating data representative of features of a curved object during examination of an object.

Fig. 5 is a schematic illustration of a method of obtaining data representative of features of an object.

Detailed description

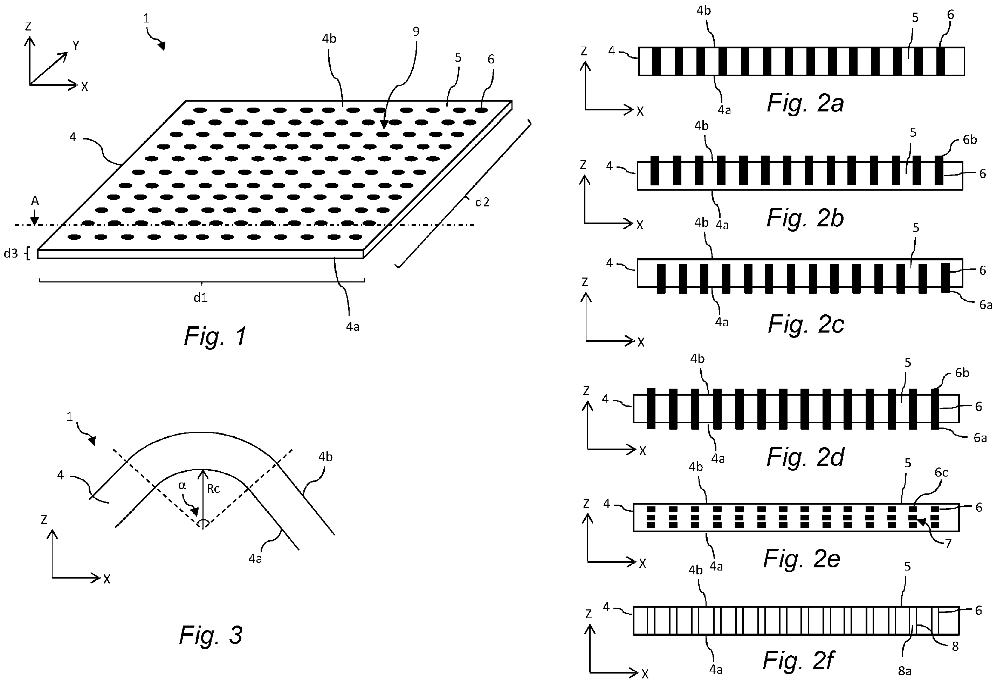

Fig. 1 shows a schematic example of an acoustic coupling interface 1 according to the present disclosure. The interface 1 is in the form of a sheet 4 that extends in an X-Y plane, with a thickness extending in a Z-direction perpendicular to both X and Y directions. The sheet 4 has a first planar surface 4a and a second planar surface 4b opposite the first planar surface 4a, and since the interface 1 is for use between a flexible ultrasound transducing device 2 and an object, such as a curved object 3, one of the planer surfaces may during use face the object 3 that is examined whereas the opposite planar surface faces the ultrasound transducing device. Consequently, the sheet 4 has a first planar surface 4a arranged for contacting said flexible ultrasound transducing device 2 and a second planar surface 4b, opposite said first surface 4a, arranged for contacting said curved object 3 to be examined.

The sheet 4 has in this example a rectangular or quadratic shape with a length d1 in the X direction of about 5-20 cm, such as about 10 cm and a length d2 in the Y-direction of about 5-20 cm, such as about 10 cm. The sheet is further thin in relation to the surface areas of the first 2a and second 2b planar surfaces, such as having a thickness d3 in the Z-direction of about 0.1 mm -1.0 mm. Thus, the first 4a and second 4b planar surfaces may have a length and width that both are at least fifty times the thickness of the sheet.

Moreover, the sheet 4 comprises a bulk material 5 and a plurality of acoustic waveguiding structures 6 arranged in the bulk material (5). In the example of Fig. 1, the acoustic waveguiding structures 6 are arranged as a two-dimensional array 9 in the bulk material 5.

The plurality of acoustic waveguiding structures 6 is for providing bidirectional coupling of ultrasound signals 7 emitted an ultrasound transducing device 2 during examination of an object 3.

Figs. 2a-f shows different embodiments of acoustic waveguiding structures 6. Figs. 2a-f are section view of a sheet 4, e.g. a section view along line A of the sheet in Fig. 1.

Fig. 2a shows a schematic embodiment of acoustic waveguiding structures 6 arranged within the bulk material 5. The waveguiding structures 6 have an elongated form extending from the first planar surface 4a to the second planar surface 4b. Thus, the waveguiding structures 6 extend in the Z direction, i.e. in the direction in which the thickness of the sheet 4 is defined. The elongated waveguiding structures, or elongated waveguides 6, may have any suitable form, such as in the form of cylinders or having convex or concave outer forms.

Fig. 2b shows a schematic embodiment of acoustic waveguiding structures 6 arranged within the bulk material 5. In this example, the waveguiding structures 6 protrude from the second planar surface 4b. Thus, the waveguiding structures 6 comprises a portion 6b that extends or protrudes out from the second planar surface 6b. The second planar surface 4b may thus comprise an array of protruding portions 6b. The protruding portions 6b may aid in keeping the contact between the acoustic coupling interface 1 with an object 3 during examination by making the second outer planar surface 4b of the sheet 4 more sticky, i.e. by increasing the friction between the interface 1 and the object 3 during examination.

The acoustic waveguiding structures 6 may also protrude from the first planar surface 4a. This example is shown in Fig. 2c, in which the waveguiding structures 6 comprises a portion 6a that extends or protrudes out from the first planar surface 6a. The first planar surface 4a may thus comprise an array of protruding portions 6a. The protruding portions 6a may aid in keeping the contact between the acoustic coupling interface 1 and a flexible ultrasound transducing device 2 during examination by making the first outer planar surface 4a of the sheet 4 more sticky, i.e. by increasing the friction between the interface 1 and the flexible ultrasound transducing device 2 during examination.

The acoustic waveguiding structures 6 may also protrude both from the first planar surface 4a and the second planar surface 4b. Such an example is shown in Fig. 2d, in which the acoustic waveguiding structures 6 both comprises a portion 6a protruding out from the first planar surface 6a and a portion 6b protruding out from the second planar surface 6b.

Having such an arrangement of protruding portions 6a and/or 6b, using a gel between interface 1 and transducer array 2, or a gel between interface and the object 3 that is examined, may be unnecessary.

Fig. 2e shows a schematic embodiment of the sheet 4 in which plurality of acoustic waveguiding structures 6 is arranged within the bulk material 5 and comprises an arrangement 7 of alternating bulk material 5 and another material 6c different from the bulk material 5. The arrangement 7 extends in the Z-direction from the first planar surface 4a to the second planar surface 4b. The material 6c other than the bulk material is thus arranged as discrete elements within the bulk 4, e.g. along imaginary straight lines extending from the first planar surface 4a to the second planar surface 4b. The size of the discrete elements and the distance between adjacent discrete elements makes the arrangement 7 work as a guiding structure for ultrasonic waves propagating through the sheet 4.

Fig. 2f shows a further schematic embodiment of a sheet 4 comprising a plurality of acoustic waveguiding structures similar to the embodiment discussed in relation to Fig. 2a above, but the acoustic waveguiding structures 6 are in the form of voids 8a arranged within the bulk material 5. Thus, the plurality of acoustic waveguiding structures 6 comprises in this example internal walls 8 in the bulk material 5 so as to define waveguiding structures in the form of voids 8a in the bulk material 5.

The acoustic waveguiding structures 6 may be of a material having a different acoustic impedance than the bulk material 5. Thus, the plurality of acoustic waveguiding structures 6 may comprise a solid material different than the bulk material 5.

The acoustic waveguiding structures 6 may be or comprise a metal or polymer, and may form three-dimensional acoustic impedance objects within the bulk material 5.

The bulk material 5 may comprise a polymer, such as polyimide (PI). The bulk material may be a flexible material so that the sheet 4 becomes flexible.

Thus, the sheet 4 may be flexible so as to form a continuous contact with a curved object 3 during operation of a flexible ultrasound transducing device 2. As an example, the sheet 4 may have a bending flexibility such that the surface profiles of both the first 4a and second b planar surfaces are altered during examination. The second planar surface, which is the surface in contact with or closest to the object being examined, may conform to the curved object 3 when the sheet 4 is in contact with a curved object 3 during examination. However, the sheet 4 may be flexible enough so that also the first planar surface 4a may conform to the curved object during examination. Consequently, the acoustic coupling interface 1 may facilitate transfer of the surface profile of the object 3 being examined to a flexible ultrasound transducing device during examination.

Fig. 3 illustrates how the flexibility of the sheet 4 may be measured. In analogy with what is shown in Fig. 1, the first 4a and second 4b planar surfaces extend in an X and Y direction and the sheet 4 has a thickness extending in a Z direction that is perpendicular to the X and Y directions. The sheet may have a flexibility and such that it may be bent in positive or negative Z direction with a bend angle ($\alpha$) that is at least 30 degrees without breaking. The thickness of the sheet 4 may in combination with the material of the bulk material, be the most important factor influencing the flexibility of the sheet 4. When bending the sheet 4, the "breaking" may refer to cracks appearing on the outer surface during bending, i.e. the surface having an area under tension during the bending. In the Example shown in Fig. 3, this area would be the surface area of the second planar surface 2b.

Further, the radius of curvature Rc may be less than 5 cm without the sheet 4 breaking. The radius of curvature may be the inside curvature during bending. Thus, in Fig. 3, the radius of curvature is measured on the first planar surface 3a since the sheet 4 is bent in negative Z - direction.

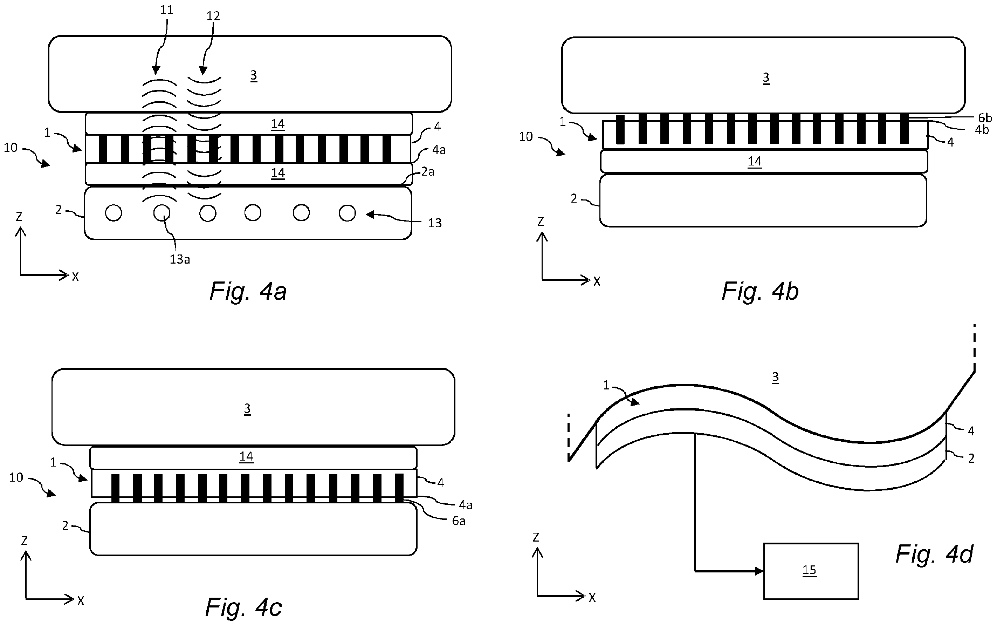

Figs. 4a-4d show different schematic and illustrative embodiments of a system 10 for creating data representative of features of an object 3.

As shown in Fig. 4a, the system 10 comprises a flexible ultrasound transducing device 2. This device 2 comprises an array 13 of individual ultrasound transducing elements 13a. The ultrasound transducing device 2 is for both emitting ultrasonic waves 11 and for receiving echo signals 12 from the object 3 being examined. The individual ultrasound transducing elements 13a of the array 13 may be micromachined ultrasound transducers (MUT), which are known in the art. Such element 13a may be formed by processing a small drum with a suspended membrane on top of a small cavity. The dimensions of this cavity in combination with the stiffness of the membrane will determine the resonance frequency of a particular MUT. The MUT may be driven by the piezoelectric effect, forming a pMUT, which functions by applying an AC electric field at the resonance frequency across a piezoelectric material to generate a stress difference between the piezo-electric material and the membrane. This will induce a vibration and the emission of an acoustical wave. Typical frequencies are in the range of 50 kHz to 20 MHz. This translates into wavelengths ranging from 1 cm down to <100 µm.

Applications that use beam-forming to create a focal spot in emission or to image a small spot in receiving, may require larger arrays of ultrasound transducing elements 13a working together.

The system 10 further comprises an acoustic coupling interface 1 as disclosed herein above. The interface 10 is removably attached onto an outer surface 2a of the transducer 2 between the transducer 2 and the object 3 being examined. The object 13 may be a part of a body, such as an arm or a leg. The acoustic coupling interface 1 thus provides for bidirectional coupling such that the ultrasound signals 11 emitted by the transducer 2 are transmitted into the object 3 and the resultant echo signals 12 from the object 3 are transmitted back to the array 13a of the transducer 2.

The ultrasound transducing elements 13 a are configured for generating ultrasonic energy propagating along a main transducer axis parallel to Z-axis, and the flexible ultrasound transducing device 2 may comprise a first outer surface 2a facing the curved object 3 during examination. This first outer surface 2a of the transducer 2 thus has a normal vector that is parallel to the main transducer axis, and the acoustic coupling interface 1 is arranged between the object 3 and the transducer 2 with the first outer planar surface 4a of the sheet 4 facing the first outer surface 2a of the transducer 2. In the embodiments illustrated in Fig. 2a, a gel 14 is applied between the flexible ultrasound transducer 2 and the acoustic coupling interface 1 and between the acoustic coupling interface 1 and the object 3 being examined.

Fig. 4b shows an embodiment of the system 10 in which the waveguiding structures 6 of the acoustic coupling interface 1 has protruding portions 6b on the second planar surface 4b of the sheet 4. This provides for ultrasonic examination of the curved object 3 without having a gel between the acoustic coupling interface and the object 3. The protrusions 6b may facilitate adhesion of the interface 1 and the whole system 10 to the object 3 being examined. It may also be beneficial is certain applications to have a dry contact between object 3 and the system 10.

Fig. 4c shows an embodiment of the system 10 in which the waveguiding structures 6 of the acoustic coupling interface 1 has protruding portions 6a on the first planar surface 4a of the sheet 4. This provides for ultrasonic examination of the curved object 3 without having a gel between the acoustic coupling interface and the ultrasonic transducer. In analogy with the embodiment shown in Fig. 4b, the protrusions 6a may aid in the adhesion of the interface 1 to the flexible ultrasound transducer 2. It may be beneficial is certain applications to have a dry contact between the ultrasound transducer 2 and a disposable acoustic coupling interface 1.

Fig 4d shows the use of the system 10 for examining a curved object 3. As illustrated in Fig. 4d, the flexibility of the sheet 4 makes it possible for the whole interface 1 to conform to the curved surface of the object 3 during examination, Further, the flexibility of the acoustic coupling interface 1 also makes it possible for the flexible ultrasound transducer 2 to conform to the curvature of the curved object 2 during examination. In this embodiment, neither a gel between the acoustic coupling interface 1 and the ultrasonic transducer 2 nor a gel between the acoustic coupling interface and the object 3 is used. Consequently, the acoustic coupling interface provides for a dry contact during examination and thus the exclusion of a gel, which may be practical benefit in various applications.

The system as disclosed in Figs 4a-4c is for creating data representative of features of an object 3. The data obtained may for example be used by a control unit 15 in the system 10 for creating an image of the internal of the object 3. The control unit 15 may comprise a communication interface, such as a transmitter/receiver, via which it may receive and transmit data from and to the ultrasound transducer 2. The control unit 15 may comprise a processing unit, such as a central processing unit, for calculating image parameters using the data obtained from the ultrasound transducer 2. Such a processing unit may be configured to execute computer code instructions which for instance may be stored on a memory.

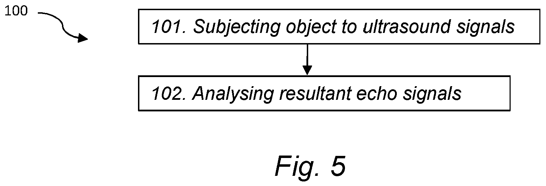

Fig. 5 schematically illustrates a method 100 of obtaining data representative of features of an object. The method 100 comprises subjecting 101 the object to ultrasound signals using a system 10 as disclosed herein above for creating data representative of features of an object 3.

Further, the method 100 comprises analysing (102) the resultant echo signals from the object 3 and thereby obtaining data representative of features of said object based on the resultant echo signals. The analyses may for example be performed by a control unit as discussed in relation to Fig. 4d above.

The step 101 of subjecting the object to ultrasound signals may be performed with a direct contact of the acoustic coupling interface 1 and the object 3, such as shown in Fig. 4b, with a direct contact of the acoustic coupling interface 1 and the flexible ultrasound transducing device 2, such as shown in Fig. 4c above, or with a direct contact of the acoustic coupling interface 1 with the object 3 and the ultrasound transducer 2, such as shown in Fig. 4d.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. An acoustic coupling interface (1) for use between a flexible ultrasound transducing device (2) and a curved object (3) to be examined; wherein said interface (1) is in the form of a sheet (4) having a bending flexibility that permits the sheet (4) to form a continuous contact with said curved object (3) during operation of the flexible ultrasound transducing device (2), and wherein said sheet (4) comprises a bulk material (5), **characterized in that** it comprises a plurality of acoustic waveguiding structures (6) arranged in said bulk material (5), wherein the plurality of acoustic waveguiding structures (6) is for providing bidirectional coupling of ultrasound signals (7) emitted by the ultrasound transducing device (2), wherein the acoustic waveguiding structures (6) are configured as an acoustic lens for an ultrasonic wave emitted by the ultrasonic transducing device (2) and for the echo signal received from the curved object (3) to be examined; wherein the plurality of acoustic waveguiding structures (6) comprises waveguiding structures having an elongated form and thus form pillars extending through part or the whole bulk material.

2. An acoustic coupling interface (1) according to claim 1, wherein said sheet has a first planar surface (4a) arranged for contacting said flexible ultrasound transducing device (2) and a second planar surface (4b), opposite said first surface (4a), arranged for contacting said curved object (3) to be examined, and wherein said sheet (4) has a bending flexibility such that the surface profiles of both the first (4a) and second (4b) planar surfaces are altered with the second planar surface (4b) conforming to the curved object (3) when the sheet (4) is in contact with said curved object (3) during operation.

3. An acoustic coupling interface (1) according to claim 2, wherein the first (4a) and second (4b) planar surfaces have a length and width that both are at least five times the thickness of the sheet.

4. An acoustic coupling interface (1) according to any previous claim, wherein the sheet (4) has a bending flexibility that permits the sheet to be bent with a radius of curvature (Rc) that is less than 5 cm.

5. An acoustic coupling interface (1) according to any one of claims 2-4, wherein the plurality of acoustic waveguiding structures (6) comprises an arrangement (7) of alternating bulk material (5) and another material (6c) different from the bulk material (5), said arrangement (7) extending from the first planar surface (4a) to the second planar surface (4b).

6. An acoustic coupling interface (1) according to any one of claims 2-5, wherein the plurality of acoustic waveguiding structures (6) comprises waveguiding structures extending from the first planar surface (4a) to the second planar surface (4b).

7. An acoustic coupling interface (1) according to any one of claims 1-6, wherein the plurality of acoustic waveguiding structures (6) comprises a solid material different than the bulk material (5).

8. An acoustic coupling interface (1) according to claim 6, wherein the plurality of acoustic waveguiding structures (6) protrudes out from the first (4a) and/or second (4b) planar surface.

9. An acoustic coupling interface (1) according to any one of claims 1-5, wherein the plurality of acoustic waveguiding structures (6) comprises internal walls (8) in the bulk material (5) so as to define waveguiding structures in the form of voids (8a) in the bulk material (5).

10. An acoustic coupling interface (1) according to any previous claim, wherein the bulk material (5) comprises a polymer.

11. A system (10) for creating data representative of features of a curved object (3) comprising

    a flexible ultrasound transducing device (2), and an acoustic coupling interface (1) according to any one of claims 1-10 configured to be removably attached to said ultrasound transducing device (2) such that ultrasound signals (11) emitted by the transducing device (2) are transmitted into said object (3) and resultant echo signals (12) from the object (3) are transmitted back to the ultrasound transducing device (2).

12. A system (10) according to claim 11, wherein said flexible ultrasound transducing device (2) comprises an array (13) of ultrasound transducers (13a).

13. A method (100) of obtaining data representative of features of a curved object comprising

    - subjecting (101) the object to ultrasound signals using a system according to any one of claims 11-12;
    - analysing (102) the resultant echo signals from the object and thereby obtaining data representative of features of said object based on the resultant echo signals.

14. A method according to claim 13, wherein the step of subjecting the object to ultrasound signals is performed with a direct contact of the acoustic coupling interface and the object and/or a direct contact of the acoustic coupling interface and the flexible ultrasound transducing device.

**Patentansprüche**

1. Akustische Kopplungsschnittstelle (1) zur Verwendung zwischen einer flexiblen Ultraschallwandler-Vorrichtung (2) und einem zu untersuchenden gekrümmten Objekt (3); wobei die Schnittstelle (1) in der Form einer Folie (4) vorliegt, die eine Biegeflexibilität aufweist, die es der Folie (4) erlaubt, einen durchgehenden Kontakt mit dem gekrümmten Objekt (3) während des Betriebs der flexiblen Ultraschallwandler-Vorrichtung (2) zu bilden, und wobei die Folie (4) ein Grundmaterial (5) umfasst, **dadurch gekennzeichnet, dass** sie eine Vielzahl von akustischen Wellenleiterstrukturen (6) umfasst, die in dem Grundmaterial (5) angeordnet sind, wobei die Vielzahl von akustischen Wellenleiterstrukturen (6) zum Bereitstellen einer bidirektionalen Kopplung von Ultraschallsignalen (7), die von der Ultraschallwandler-Vorrichtung (2) emittiert werden, bestimmt ist, wobei die akustischen Wellenleiterstrukturen (6) als eine akustische Linse für eine Ultraschallwelle, die von der Ultraschallwandler-Vorrichtung (2) emittiert wird, und für das Echosignal, das von dem zu untersuchenden gekrümmten Objekt (3) empfangen wird, konfiguriert sind; wobei die Vielzahl von akustischen Wellenleiterstrukturen (6) Wellenleiterstrukturen umfassen, die eine längliche Form aufweisen und somit Säulen bilden, die sich durch einen Teil oder das ganze Grundmaterial hindurch erstrecken.

2. Akustische Kopplungsschnittstelle (1) nach Anspruch 1, wobei die Folie eine erste planare Oberfläche (4a), die zum Kontaktieren der flexiblen Ultraschallwandler-Vorrichtung (2) angeordnet ist, und eine zweite planare Oberfläche (4b) gegenüber der ersten Oberfläche (4a), die zum Kontaktieren des zu untersuchenden gekrümmten Objekts (3) angeordnet ist, aufweist, und wobei die Folie (4) eine derartige Biegeflexibilität aufweist, dass die Oberflächenprofile sowohl der ersten (4a) als auch der zweiten (4b) planaren Oberfläche geändert sind, wenn sich die zweite planare Oberfläche (4b) an das gekrümmte Objekt (3) anschmiegt, wenn die Folie (4) mit dem gekrümmten Objekt (3) im Betrieb in Kontakt steht.

3. Akustische Kopplungsschnittstelle (1) nach Anspruch 2, wobei die erste (4a) und die zweite (4b) planare Oberfläche eine Länge und eine Breite aufweisen, die beide mindestens fünfmal die Dicke der Folie sind.

4. Akustische Kopplungsschnittstelle (1) nach einem der vorhergehenden Ansprüche, wobei die Folie (4) eine Biegeflexibilität aufweist, die es der Folie ermöglicht, sich mit einem Krümmungsradius (Rc) von weniger als 5 cm zu biegen.

5. Akustische Kopplungsschnittstelle (1) nach einem der Ansprüche 2 bis 4, wobei die Vielzahl von akustischen Wellenleiterstrukturen (6) eine Anordnung (7) von abwechselndem Grundmaterial (5) und einem anderen Material (6c), das anders als das Grundmaterial (5) ist, umfasst, wobei sich die Anordnung (7) von der ersten planaren Oberfläche (4a) zu der zweiten planaren Oberfläche (4b) erstreckt.

6. Akustische Kopplungsschnittstelle (1) nach einem der Ansprüche 2 bis 5, wobei die Vielzahl von akustischen Wellenleiterstrukturen (6) Wellenleiterstrukturen umfasst, die sich von der ersten planaren Oberfläche (4a) zu der zweiten planaren Oberfläche (4b) erstrecken.

7. Akustische Kopplungsschnittstelle (1) nach einem der Ansprüche 1 bis 6, wobei die Vielzahl von akustischen Wellenleiterstrukturen (6) ein anderes festes Material als das Grundmaterial (5) umfasst.

8. Akustische Kopplungsschnittstelle (1) nach Anspruch 6, wobei die Vielzahl von akustischen Wellenleiterstrukturen (6) aus der ersten (4a) und/oder der zweiten (4b) planaren Oberfläche vorsteht.

9. Akustische Kopplungsschnittstelle (1) nach einem der Ansprüche 1 bis 5, wobei die Vielzahl von akustischen Wellenleiterstrukturen (6) interne Wände (8) in dem Grundmaterial (5) umfasst, um Wellenleiterstrukturen in Form von Hohlräumen (8a) in dem Grundmaterial (5) zu definieren.

10. Akustische Kopplungsschnittstelle (1) nach einem der vorhergehenden Ansprüche, wobei die Grundmaterial (5) ein Polymer umfasst.

11. System (10) zum Erstellen von Daten, die für Merkmale eines gekrümmten Objekts (3) repräsentativ sind, umfassend

    eine flexible Ultraschallwandler-Vorrichtung (2), und
    eine akustische Kopplungsschnittstelle (1) nach einem der Ansprüche 1 bis 10, die dazu konfiguriert ist, abnehmbar an der Ultraschallwandler-Vorrichtung (2) angebracht zu sein, so dass die Ultraschallsignale (11), die von der Wandlervorrichtung (2) emittiert werden, in das Objekt (3) übertragen werden und sich ergebende Echosignale (12) von dem Objekt (3) zurück an die Ultraschallwandler-Vorrichtung (2) übertragen werden.

**12.** System (10) nach Anspruch 11, wobei die flexible Ultraschallwandler-Vorrichtung (2) eine Anordnung (13) von Ultraschallwandlern (13a) umfasst.

**13.** Verfahren (100) zum Erzielen von Daten, die Merkmale eines gekrümmten Objekts darstellen, umfassend

- Aussetzen (101) des Objekts an Ultraschallsignale unter Verwendung eines Systems nach einem der Ansprüche 11 bis 12;
- Analysieren (102) der sich ergebenden Echosignale von dem Objekt und dadurch Erzielen von Daten, die für Merkmale des Objekts repräsentativ sind, basierend auf den sich ergebenden Echosignalen.

**14.** Verfahren nach Anspruch 13, wobei der Schritt des Aussetzens des Objekts an Ultraschallsignale mit einem direkten Kontakt der akustischen Kopplungsschnittstelle und dem Objekt und/oder einem direkten Kontakt der akustischen Kopplungsschnittstelle und der flexiblen Ultraschallwandler-Vorrichtung erfolgt.


**Revendications**

**1.** Interface de couplage acoustique (1) destinée à être utilisée entre un dispositif de transduction ultrasonore flexible (2) et un objet courbe (3) à examiner ; Dans laquelle ladite interface (1) se présente sous la forme d'une feuille (4) présentant une flexibilité de flexion qui permet à une feuille (4) d'établir un contact continu avec ledit objet incurvé (3) pendant le fonctionnement du dispositif de transduction ultrasonore flexible (2) et dans lequel ladite feuille (4) est constituée de un matériau massif (5), **caractérisé en ce qu'**elle comprend une pluralité de structures de guidage d'ondes acoustiques (6) disposées dans ledit matériau massif (5), dans lequel la pluralité de structures de guidage d'ondes acoustiques (6) assurent un couplage bidirectionnel des signaux ultrasonores (7) émis par le dispositif de transduction ultrasonore (2), dans lequel les structures de guidage d'ondes acoustiques (6) sont configurées comme une lentille acoustique pour une onde ultrasonore émise par le dispositif de transduction ultrasonore (2) et pour le signal d'écho reçu de l'objet incurvé (3) à examiner, dans lequel la pluralité de structures de guidage d'ondes acoustiques (6) comprennent des structures de guidage d'ondes présentent une forme allongée et forment ainsi des piliers traversant tout ou partie du matériau massif.

**2.** Interface de couplage acoustique (1) selon la revendication 1, dans laquelle ladite feuille présente une première surface plane (4a) destinée à entrer en contact avec ledit dispositif de transduction ultrasonore flexible (2) et une deuxième surface plane (4b), opposée à ladite première surface (4a), destinée à entrer en contact avec ledit objet courbe (3) à examiner et dans lequel ladite a feuille (4) présente une flexibilité de flexion telle que les profils de surface des première (4a) et deuxième (4b) surfaces planes sont modifiés, la deuxième surface plane (4b) s'adaptant à l'objet courbe (3) lorsque la feuille (4) est en contact avec ledit objet courbe (3) pendant le fonctionnement.

**3.** Interface de couplage acoustique (1) selon la revendication 2, dans laquelle les première (4a) et deuxième (4b) surfaces planes ont une longueur et une largeur au moins égales à cinq fois l'épaisseur de la feuille.

**4.** Interface de couplage acoustique (1) selon une quelconque des revendications précédentes, dans laquelle la feuille (4) présente une flexibilité de flexion lui permettant d'être pliée avec un rayon de courbure (Rc) qui est inférieur à 5 cm.

**5.** Interface de couplage acoustique (1) selon une quelconque des revendications 2 à 4, dans laquelle la pluralité de structures de guidage d'ondes acoustiques (6) comprend un agencement (7) alternant un matériau massif (5) et un autre matériau (6c) différent du matériau massif (5), ledit agencement (7) s'étendant de la première surface plane (4a) à la deuxième surface plane (4b).

**6.** Interface de couplage acoustique (1) selon une quelconque des revendications 2 à 5, dans laquelle la pluralité de structures de guidage d'ondes acoustiques (6) comprend des structures de guidage d'ondes s'étendant de la première surface plane (4a) à la deuxième surface plane (4b).

**7.** Interface de couplage acoustique (1) selon une quelconque des revendications 1 à 6, dans laquelle la pluralité de structures de guidage d'ondes acoustiques (6) est constituée d'un matériau solide différent du matériau massif (5).

**8.** Interface de couplage acoustique (1) selon la revendication 6, dans laquelle la pluralité de structures de guidage d'ondes acoustiques (6) dépasse de la première (4a) et/ou de la deuxième (4b) surface plane.

**9.** Interface de couplage acoustique (1) selon une quelconque des revendications 1 à 5, dans laquelle la pluralité de structures de guidage d'ondes acoustiques (6) comprend des parois internes (8) dans le matériau massif (5) de manière à définir des structures de guidage d'ondes sous forme de vides (8a) dans le matériau massif (5).

**10.** Interface de couplage acoustique (1) selon une quelconque des revendications précédentes, dans laquelle le matériau massif (5) est constitué d'un polymère.

**11.** Système (10) de création de données représentatives des caractéristiques d'un objet courbe (3), comprenant un dispositif de transduction ultrasonore flexible (2) et
une interface de couplage acoustique (1) selon une quelconque des revendications 1 à 10, configurée pour être fixée de manière amovible audit dispositif de transduction ultrasonore (2) de sorte que les signaux ultrasonores (11) émis par le dispositif de transduction (2) soient transmis à l'objet (3) et que les signaux d'écho résultants (12) de l'objet (3) soient retransmis au dispositif de transduction ultrasonore (2).

**12.** Système (10) selon la revendication 11, dans lequel ledit dispositif de transduction ultrasonore flexible (2) comprend un réseau (13) de transducteurs ultrasonores (13a).

**13.** Procédé (100) d'obtention de données représentatives des caractéristiques d'un objet courbe, comprenant :

- la soumission (101) de l'objet à des signaux ultrasonores à l'aide d'un système selon une quelconque des revendications 11 et 12 ;
- l'analyse (102) des signaux d'écho résultants de l'objet et l'obtention de données représentatives des caractéristiques dudit objet à partir des signaux d'écho résultants.

**14.** Procédé selon la revendication 13, dans lequel l'étape de soumission de l'objet à des signaux ultrasonores est réalisée par contact direct de l'interface de couplage acoustique avec l'objet et/ou par contact direct de l'interface de couplage acoustique avec le dispositif de transduction ultrasonore flexible.

Fig. 2a

Fig. 2b

Fig. 2c

Fig. 2d

Fig. 2e

Fig. 2f

Fig. 1

Fig. 3

Fig. 4a

Fig. 4b

Fig. 4c

Fig. 4d

100

101. Subjecting object to ultrasound signals

102. Analysing resultant echo signals

*Fig. 5*

## REFERENCES CITED IN THE DESCRIPTION

**Patent documents cited in the description**

- US 20180168544 A
- US 20190046158 A1
- US 5680863 A1
- US 20100011865 A1